# EUROPEAN PATENT SPECIFICATION

(11) **EP 2 546 247 B1**
(45) Date of publication and mention of the grant of the patent: **29.07.2015**
(21) Application number: 12175772.8
(22) Date of filing: 10.07.2012
(51) Int. Cl.: C07D 401/04, A61K 31/506, A61P 35/00

(54) **Imatinib mesylate preparation procedure**
Verfahren zur Herstellung von Imatinibmesylat
Procédure de préparation de mésylate d'imatinib

(30) Priority: 14.07.2011 IT MI20111309
(43) Date of publication of application: 16.01.2013
(73) Proprietor: F.I.S.- Fabbrica Italiana Sintetici S.p.A., 36075 Alte di Montecchio Maggiore (VI) (IT)
(72) Inventor: Grendele, Ennio, I-36078 Valdagno, VICENZA (IT); Galvagni, Marco, I-37131 VERONA (IT)
(74) Representative: Leganza, Alessandro

(56) References cited:
- WO-A1-2006/024863
- WO-A1-2011/099039
- WO-A1-2011/108953
- WO-A2-2009/147626
- JP-A- 4 266 931
- US-A1- 2009 264 438

## Description

### Background of the invention

The subject of the present invention is a procedure for preparing Imatinib mesylate.

### State of the art

Imatinib mesylate, compound of formula (I), is an important active ingredient used to treat chronic myelogenous leukaemia.

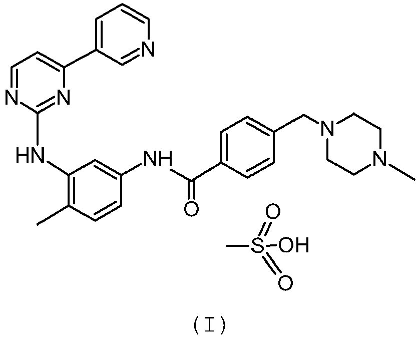

Its preparation is described for the first time in Novartis' EP 564409 and subsequently in EP998473 where the preparations of Imatinib mesylate alpha and beta polymorphic form are described. An interesting and cheap procedure for the preparation of the key intermediate is described in WO2008/059551 in the name of F.I.S. Fabbrica Italiana Sintetici S.p.A. Also described are other preparations of polymorphic forms of Imatinib mesylate, in particular the alpha and beta forms, in WO2006/024863, US2006/0223816, WO2008/150481 and WO2009/151899. In such a literature an important and far from negligible problem in the pharmaceutical field is never mentioned however, which is that relating to the residual solvents. These do in fact represent impurities of the active ingredient and of the formulated product like typical organic impurities and as such, in line with the ICH guidelines and related national and European guidelines, must be quantified and comply with the relative limits.

### Summary of the invention

The problem addressed by the present invention is therefore that of providing a procedure for the preparation of Imatinib mesylate of formula (I) having a residual solvent content in conformity with current ICH guidelines and pharmacopoeias.

Such a problem is solved by a procedure for the synthesis of Imatinib mesylate as outlined in the attached claims, the definitions of which form an integral part of the present description.

Further characteristics and advantages of the process according to the invention include but are not limited to those indicated in the description of examples of preferred embodiments provided below.

### Brief description of the figures

By way of example:
Figure 1 exemplifies the rotor-stator principle;
Figure 2 exemplifies an embodiment of the present invention on laboratory scale with an appropriate disperser;
Figure 3 exemplifies a part of an industrial disperser wherein the three in-series rotor-stator combinations can be seen, which permit the breaking up into small pieces and the homogenization of the product in suspension.

### Detailed description of the invention

The present invention concerns a procedure for the preparation of Imatinib mesylate of formula (I): 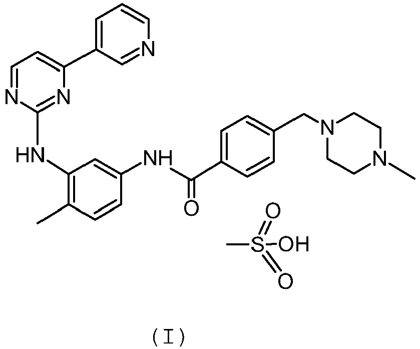 having a residual solvent content in compliance with the guidelines of the ICH (International Conference of Harmonization) Q3C(R4) (Impurities: guideline for Residual Solvents) published in February 2009. A particular reference is made with respect to the solvents classified in Class 3 for which a residual solvent limit of 5000 ppm is required.

Experimentally repeating the preparatory methods described in the state of the art, though obtaining the product in the alpha and beta polymorphic forms, the problem of the residual solvent content, always outside the ICH limits, was still present. The problem was evident even when different types of solvents were used, e.g., alcohols, ketones, acetates, ethers, etc.. It was then found that by treating a suspension of Imatinib mesylate in an organic solvent with a disperser or crusher comprising one or more stator combinations and one or more rotors, Imatinib mesylate can be obtained having residual solvents in compliance with the ICH guidelines and pharmacopoeias.

By preparing Imatinib mesylate, in both alpha and beta polymorphic form, e.g., crystallizing or recrystallizing it from n-butanol, a product is obtained containing over 17000 ppm of residual n-butanol even if the product is dried at 60°C under vacuum for 48 hours. By drying the product at 120°C under vacuum for 24 hours or under nitrogen flow at 90°C, the product still contains 9000-10000 ppm of residual n-butanol which is much higher than the 5000 ppm of the ICH limit. Totally similar problems, with the same levels of residual solvents, can be observed by crystallizing the product, for example, from ethanol, isopropanol, MEK or MTBE.

Using the procedure of the present invention, employing for example n-butanol, even by drying the product at just 60°-80°C for 10-12 hours, Imatinib mesylate is obtained having less than 5000 ppm of n-Butanol, normally around 3000 ppm and in any case always above 1000 ppm and below 5000 ppm, and therefore as a matter of fact always in compliance with the current ICH limits for such solvent.

A possible scientific explanation of the effect of the present invention has been found by determining that the product crystallizes by combining solvent molecules inside the crystal aggregates. It has thus also been confirmed by means of DSC and TGA measurements that the solvent does not enter the crystal lattice to form a solvated species, rather it is simply solvent combined in the crystal aggregate which is nevertheless very difficult to remove or bring it within permitted limits using conventional drying methods. On an industrial scale, the problem of drying the product in such a way as to lead to a product of pharmaceutically acceptable grade has dragged on for quite a long time without any major and industrially pursuable solutions being found.

The procedure of the present invention is therefore achieved by contemplating a treatment of a suspension of Imatinib mesylate with a crusher or disperser able to break down the particles of product in suspension. The grinding treatment carried out on the solid dried or partially dried product does not produce the same results inasmuch as the quantities of residual solvents remain high.

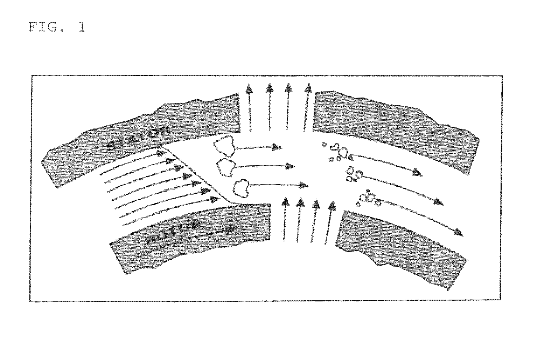

Appliances have been available for a long time at both laboratory level and industrial level which are able to perform this product dispersion procedure, able to split up and distribute the solid in the liquid phase, thereby obtaining a homogeneous suspension. The principle on which these appliances are based is called rotor-stator principle and can be exemplified as in figure 1 and summed up as follows. The high number of rpm of the rotor axially sucks up the fluid into the dispersion head and is then pushed radially through the slots of the rotor-stator. The strong accelerating forces apply very strong tension and thrust forces on the material. In the slot between the rotor and the stator, a strong turbulence is also produced that causes the suspension to mix in the best possible way. These types of appliances can be called dispersers, crushers, homogenizers or, simply, mixers.

The crusher/disperser of the present invention comprises one or more stator combinations and one or more rotors and operates at between 1000 and 100,000 rpm.

For laboratory applications, the best tip speed of the rotor-stator system is around 6 - 30 m/s, preferably 23 m/s and rotor ranges are used of between 3000 and 30,000 rpm. The Imatinib mesylate is therefore treated with such tip speed and rotor rpm values. In the case of industrial applications, speeds of up to 100,000 rpm can even be reached, even though, especially for very large scales, rotor speeds of 1000-2000 rpm are normally applied.

For laboratory scale applications, 8000-10000 rpm give satisfactory results. Preferably, ranges between 1000 and 10000 rpm are therefore generally used.

For laboratory applications in general, a treatment time of just a few minutes is enough to achieve the required end fineness. Longer treatment times only improve the fineness of the materials to a minor extent and consequently 15-minute treatments are normally enough.

A piece of laboratory equipment suitable for preparations in accordance with the present invention is the IKA ULTRA-TURRAX T 25 digital.

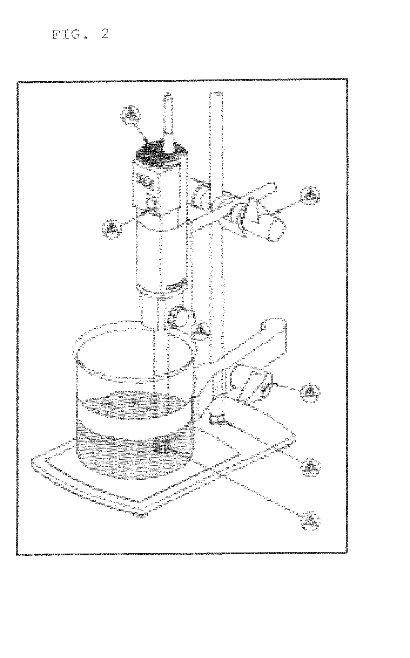

In this appliance (Figure 2) there is just one rotor-stator combination.

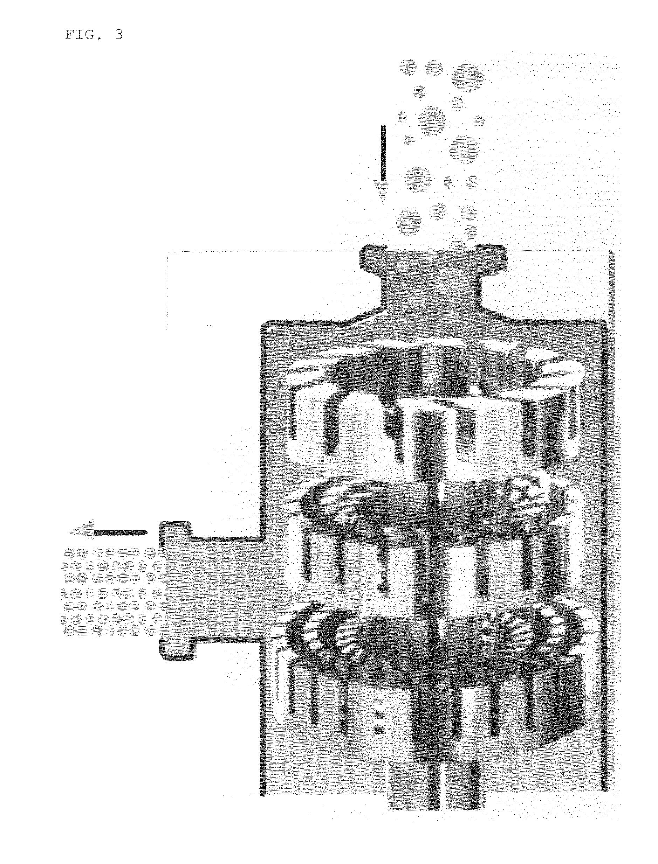

For industrial applications, the IKA® Dispax Reactor model DR 2000/10 Disperser is particularly effective inasmuch as it has a 3-stage rotor-stator system consisting of 3 rotor-stator combinations in series the one to the other (see Figure 3). Such industrial appliances also take the name of "wet mill".

For laboratory-scale preparations, the procedure is performed with treatment times between 5 and 60 minutes, preferably for about 15 minutes.

The organic solvent in which the Imatinib mesylate can be conveniently suspended or is obtained is chosen from among alcohols, ketones, acetates and ethers. The Imatinib mesylate is preferably suspended in a C1-C5 alcohol and therefore in an alcohol chosen from among methanol, ethanol, n-propanol, isopropanol, n-butanol, sec-butanol, isobutanol, tert-butanol, n-pentanol, 2-ethyl-1-propanol, 2-methyl-1-butanol, 3-methyl-1-butanol, isopentanol, sec-pentanol, 2,2-dimethyl-1-propanol. Preferably, the procedure of the present invention is conducted by preparing a suspension of Imatinib mesylate in ethanol or isopropanol or n-butanol.

Still more preferably, the procedure of the invention is conducted by preparing a suspension of Imatinib mesylate in n-butanol. This solvent has the advantage of allowing a slow crystallization, for example differently from isopropanol and the other lower alcohols, wherein the crystallization of the product is very fast. This advantage results in the n-butanol being a solvent which permits, depending on the crystallization conditions, obtaining the alpha or the beta form of Imatinib mesylate.

The procedure of the present invention therefore allows preparing Imatinib mesylate of formula (I): 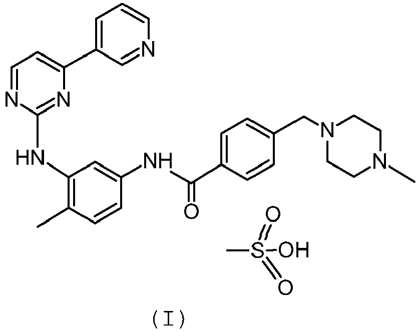 comprising between 1000 and 5000 ppm of ethanol or propanol or butanol or pentanol. In particular, the propanol is chosen from between n-propanol and isopropanol and the butanol is chosen from among nbutanol, isobutanol, sec-butanol and tert-butanol and the pentanol is chosen among n-pentanol, isopentanol and sec-pentanol.

In particular, with the process of the present invention by using n-butanol it is possible to obtain Imatinib mesylate having a residual content of such a solvent comprised between 1000 and 5000 ppm and thus in conformity with the present ICH limits and preferably between 2000 and 4000 ppm, in alfa or beta crystalline form.

In a preferred embodiment, Imatinib mesylate of formula (I) contains between 2000 and 4000 ppm of n-butanol.

Furthermore, the Imatinib mesylate is obtained in both alpha and beta polymorphic form (according to the characterization provided by the originator in EP998473).

The procedure of the present invention can be applied both during product crystallization and during a subsequent product re-crystallization or crushing stage.

The content of residual solvents is an essential characteristic for pharmaceutical products, whether these are active ingredients or formulated products, inasmuch as compliance with appropriate limits is obligatory with reference to applicable regulations on pharmaceutical products.

Moreover, Imatinib mesylate obtained with the process of the present invention by using n-butanol as a crystallization or recrystallization solvent does not contain the genotoxic impurity n-butylmesylate, whose amount is less than the detectability threshold of 0.1 ppm.

The Imatinib mesylate prepared according to the procedure of the present invention can therefore be conveniently formulated with one or more acceptable pharmaceutical excipients. The formulated product can therefore be used in medicine and, specifically, can therefore be used to cure chronic myelogenous leukaemia.

### EXPERIMENTAL PART

### Example 1 - Synthesis of Imatinib mesylate (I) alpha form - illustrative of the invention.

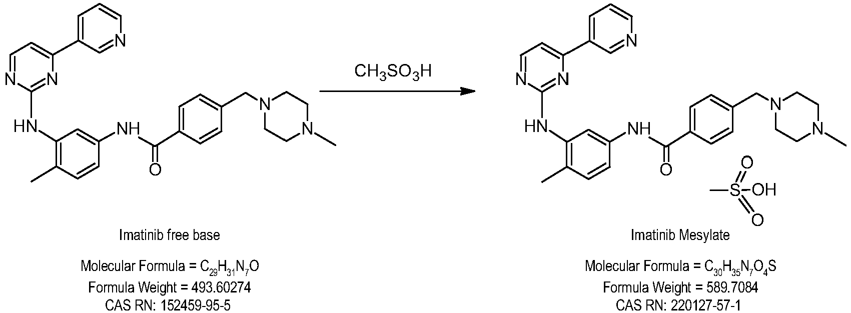

In a 20-litre steel vessel 400.0 g of Imatinib base (prepared according to example 21 of EP0564409)and 8000 ml of n-butanol are loaded under nitrogen flow and this is heated under reflux. 20.0 g of Carbon eno-pc are added and the whole is shaken at reflux temperature for 15 minutes, before filtering on steryflon ptf2071 sl cartridge and conveying the filtrate by nitrogen thrust to a preheated glass reactor. A yellowish solution is obtained at pH=6.4. Afterwards, 75.8 g (51.20 ml) of methanesulphonic acid are dripped in one minute at T=80°C. A clear solution is obtained at approx. stable 4.5 pH. This is shaken and left to cool at T=70°C and primed with 5.0 g of Imatinib mesylate, alpha form. Precipitation with rapid thickening is observed. The solution is shaken for one hour and left to cool at T=20°C. This is filtered in 5 minutes and washed with 680.0 ml of n-butanol.

It is then dried in a vacuum stove at T=50°C until constant weight, and 315 g of Imatinib mesylate, alpha form are obtained with a molar yield of 65.7%.

The product contains over 17,000 ppm of residual nbutanol determined by GC-HS.

### Example 2 - Synthesis of Imatinib mesylate (I) alpha form - illustrative of the invention.

In a reactor under nitrogen 200.0 g of Imatinib mesylate, obtained according to the Example 1, and 7000 ml of n-butanol are loaded and this is heated at reflux before being filtered hot on a dicalite cake and the filtrate is left to cool down to 80°C, temperature at which 20.0 g of Imatinib mesylate, alpha form, primer are added and this is treated for 15 minutes with a high-performance IKA disperser at around 7500 rpm (as shown in figure 2). This is shaken and left to cool at T=40°C for 30-40 minutes before being filtered and washed with a little n-butanol.

The solid is then dried at 70-80°C for 10-12 hours. 174.0 g of product are obtained, equivalent to a molar yield of 79.1%. The content of residual n-butanol is around 3000 ppm.

### Example 3 - Synthesis of Imatinib mesylate (I) beta form - illustrative of the invention

Exactly the same procedure is followed as for Example 1 except that the primer is made with 5.0 g of Imatinib mesylate beta form instead of alpha form.

About 318 g of Imatinib mesylate beta form are obtained for a molar yield of 66.3%.

### Example 4 - Synthesis of Imatinib mesylate (I) beta form - illustrative of the invention

Exactly the same procedure is followed as for Example 2 except that the primer is made with 20.0 g of Imatinib mesylate beta form instead of alpha form.

190.3 g of Imatinib mesylate beta form are obtained with a molar yield of 86,5%. The content of residual n-butanol is around 3000 ppm.

### Example 5 - GC-HS Method for residual solvent determination in Imatinib Mesylate

**Chromatographic conditions:**

| | |
|---|---|
| **Column:** | Fused silica , L = 30 m, ID = 0.54 mm, FT = 3 pm |
| **Liquid phase:** | DB-624 |
| **InjectorTemp:** | 200°C |
| **Detector Temp:** | 250°C |
| **ColumnTemp:** | 40°C for 5 min., from 40°C to 90°C at 4°C/min. from 90°C to 230°C at 20°C/min, isotherm at 230°C for 6 min. |
| **Injection:** | split, split ratio 1:1* |
| **Carrier gas:** | Helium, constant flow (flow = 4.5 mL/min) * |
| **Detector:** | Flame ionization (air 400 mL/min.; Hydrogen 40 mL/min * |
| **Make up:** | Nitrogen (flow 30 mL/min.) |
| **Diluent:** | Dimethylsulphoxide |
| **Vialsize:** | 20 mL |
| **Sample Volume:** | 5 mL |

| | |
|---|---|
| * These parameters may be modified to optimize the signal or to reach the requested performance | |

**Head-space conditions:**

| | |
|---|---|
| **Oven Temperature:** | 100°C |
| **Loop Temperature:** | 200°C |
| **Transfer Line Temperature:** | 200°C |
| **Vial Equilibration Time:** | 20 min |
| **Pressurization Time:** | 0.2 min |
| **VialPressurization:** | 6 psi |
| **Loop Filling Time:** | 0.2 min |
| **Loop Equilibration Time:** | 0.1 min |
| **Injection Time:** | 1 min |
| **ShakingSpeed:** | Low |
| **GC cycle time** | 40 min |

### Example 6 - Comparative examples

The following experimental data are reported.

Further to the comparison between the first two results (the starting product is the same for all the subsequent experimental tests), the data allow to understand the difficulties on which the problem addressed by the present invention is based.

| **Test** | **n-butanol content** |
|---|---|
| Starting product used for all the tests described below - | **21184** |
| Imatinib mesylate beta form | |
| Milling on a mortar + drying in stove T=60°C for 4 days | **17900** |
| Pulping at RT in n-Butanol + drying in stove T=60°C for 4 days | 17600 |
| Pulping at RT in n- heptane + drying in stove under vacuum T=90°C for 30 h | 19000 |
| Recrystallization from EtOH + H2O | 11600 |
| Pulping in AcOEt | 22700 |
| Treatment for 24 hr under high vacuum | 17950 |
| Treatment for 24 hr at 120°C | 19000 |
| Pulping at reflux in n- heptane+ drying in stove T=60°C. | 17800 |
| Recrystallization from n-Butanol + drying at T=120°C under vacuum for 15 hr. | 7890 |

Analogous results are obtained starting from Imatinib mesylate alpha form.

It is thus evident that, even if it is amply known to the person skilled in the art that to reduce the amount of residual solvents in a product, it can be milled by reducing the particle size, this does not give significant effects in the present case with Imatinib mesylate. This latter, differently from the greatest part of active principles known to date, has an unusual tendency to keep the residual solvents, particularly alcoholic solvents; and its milling does not allow an effective reduction of their amount.

In particular, it will be appreciated how the use of the conditions forming the subject of the present invention permit obtaining Imatinib mesylate of formula (I) both in alpha and beta polymorphic form and having a residual solvent content in compliance with ICH guidelines.

## Claims

1. Process for the preparation of Imatinib mesylate of formula(I): 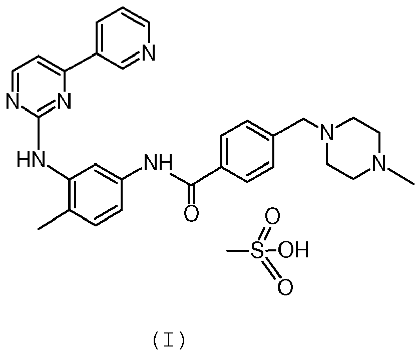 comprising the treatment of a suspension of Imatinib mesylate in an organic solvent with a crusher/disperser comprising one or more stator combinations and one or more rotors.

2. Process according to claim 1 wherein the treatment is performed at between 1000 and 100,000 rpm of the one or more rotors.

3. Process according to claim 2 wherein the treatment is performed at between 1000 and 10,000 rpm of the one or more rotors.

4. Process according to any one of the claims from 1 to 3 wherein the Imatinib mesylate is treated at a tip speed of around 23 m/s.

5. Process according to any one of the claims from 1 to 4 wherein the treatment is performed in between 5 and 60 minutes.

6. Process according to claim 5 wherein the treatment is performed for about 15 minutes.

7. Process according to any one of the claims from 1 to 6 wherein the Imatinib mesylate is in suspension in an organic solvent selected from among alcohols, ketones, acetates and ethers.

8. Process according to claim 7 wherein the Imatinib mesylate is suspended in a C1-C5 alcohol.

9. Process according to claim 8 wherein the Imatinib mesylate is suspended in Ethanol or Isopropanol or n-Butanol.

10. Use of a crusher/disperser comprising one or more stator combinations and one or more rotors for the preparation of Imatinib mesylate by means of the treatment of a suspension of Imatinib mesylate in an organic solvent with such appliance.

## Patentansprüche

1. Verfahren zur Herstellung von Imatinib Mesylat der Formel (I): 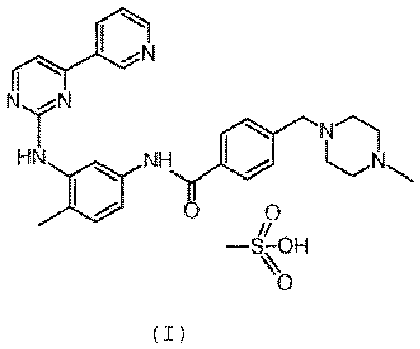 , umfassend die Behandlung einer Suspension aus Imatinib Mesylat in einem organischen Lösungsmittel mit einem Brecher/Dispergierer, der eine oder mehrere Statorkombination(en) und einen oder mehrere Rotor(en) umfasst.

2. Verfahren nach Anspruch 1, wobei die Behandlung bei zwischen 1000 und 100.000 rpm des einen Rotors oder der mehreren Rotoren durchgeführt wird.

3. Verfahren nach Anspruch 2, wobei die Behandlung bei zwischen 1000 und 10.000 rpm des einen Rotors oder der mehreren Rotoren durchgeführt wird.

4. Verfahren nach einem der Ansprüche von 1 bis 3, wobei das Imatinib Mesylat bei einer Umfangsgeschwindigkeit von etwa 23 m/s behandelt wird.

5. Verfahren nach einem der Ansprüche von 1 bis 4, wobei die Behandlung in zwischen 5 und 60 Minuten durchgeführt wird.

6. Verfahren nach Anspruch 5, wobei die Behandlung für etwa 15 Minuten durchgeführt wird.

7. Verfahren nach einem der Ansprüche von 1 bis 6, wobei das Imatinib Mesylat in einem organischen Lösungsmittel, das ausgewählt ist aus Alkoholen, Ketonen, Acetaten und Ethern, in Suspension ist.

8. Verfahren nach Anspruch 7, wobei das Imatinib Mesylat in einem C1-C5 Alkohol suspendiert ist.

9. Verfahren nach Anspruch 8, wobei das Imatinib Mesylat in Ethanol oder Isopropanol oder n-Butanol suspendiert ist.

10. Verwendung eines Brechers/Dispergierers, umfassend eine oder mehrere Statorkombination(en) und einen oder mehrere Rotor(en) zur Herstellung von Imatinib Mesylat durch die Behandlung einer Suspension aus Imatinib Mesylat in einem organischen Lösungsmittel mit solcher Vorrichtung.

## Revendications

1. Procédé de préparation de mésylate d'imatinib de formulé (1) : 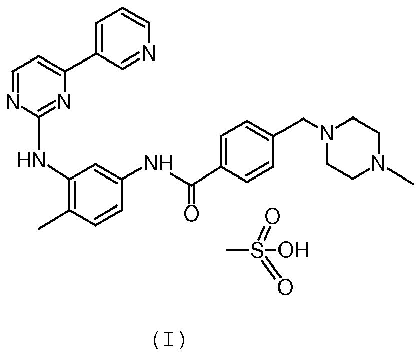 comprenant le traitement d'une suspension de mésylate d'imatinib dans un solvant organique avec un broyeur/disperseur comprenant une ou plusieurs combinaisons de stators et un ou plusieurs rotors.

2. Procédé selon la revendication 1 dans lequel le traitement est réalisé à une vitesse d'un ou plusieurs rotors comprise entre 1000 et 100 000 tr/min.

3. Procédé selon la revendication 2 dans lequel le traitement est réalisé à une vitesse d'un ou plusieurs rotors comprise entre 1000 et 10 000 tr/min.

4. Procédé selon une des revendications 1 à 3 dans lequel le mésylate d'imatinib est traité à une vitesse des lames d'environ 23 m/s.

5. Procédé selon une des revendications 1 à 4 dans lequel le traitement est réalisé dans un laps de temps compris entre 5 et 60 minutes.

6. Procédé selon la revendication 5 dans lequel le traitement est réalisé en 15 minutes environ.

7. Procédé selon une des revendications 1 à 6 dans lequel le mésylate d'imatinib est en suspension dans un solvant organique choisi dans les alcools, cétones, acétates et éthers.

8. Procédé selon la revendication 7 dans lequel le mésylate d'imatinib est en suspension dans un alcool C1-C5.

9. Procédé selon la revendication 8 dans lequel le mésylate d'imatinib est en suspension dans de l'éthanol ou de l'isopropanol ou du n-butanol.

10. Utilisation d'un broyeur/disperseur comprenant une ou plusieurs combinaisons de stators et un ou plusieurs rotors pour la préparation de mésylate d'imatinib par traitement d'une suspension de mésylate d'imatinib dans un solvant organique avec cet appareil.
